# EUROPEAN PATENT APPLICATION

(11) **EP 4 772 529 A1**
(43) Date of publication of application: **08.07.2026**
(21) Application number: 24880062.5
(22) Date of filing: 14.10.2024
(51) Int. Cl.: C07K 7/06, A61P 17/00, A61K 8/64, A61Q 19/02, A61K 38/00

(54) **PEPTIDE HAVING ACTIVITY OF SKIN WHITENING AND USES THEREOF**

(30) Priority: 16.10.2023 KR 20230138104
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR); CHO, Mihee, Seoul 06188 (KR); JEONG, Min Kyeong, Seoul 06188 (KR); JUNG, Da Won, Seoul 06188 (KR)
(74) Representative: Bandpay & Greuter
(86) International application number: PCT/KR2024/015524
(87) International publication number: WO 2025/084725

(57) **Abstract**

A peptide of the present invention exhibits the activity of skin whitening by inhibiting melanosome absorption in keratinocytes. The peptide of the present invention can be used as an active ingredient in drugs for the treatment or prevention of hyperpigmenatation diseases caused by excessive deposition of melanosomes, or as an active ingredient in cosmetics for skin whitening.

## Description

### [Technical Field]

### [Cross-citation with related applications]

This application claims the benefit of priority based on Korean Patent Application No. 10-2023-0138104 filed October 16, 2023, the entire contents of which are incorporated herein as part of this specification.

### [Technical Field]

The present invention relates to a peptide having skin whitening activity and uses thereof.

### [Background Art]

Cutaneous melanin, found in the skin, hair follicles, and eyes, plays a vital role in protecting the skin from UV rays. However, excessive melanin production can cause hyperpigmentation disorders such as melasma, freckles, and age spots, leading to psychological stress and a decline in quality of life. Melanin is synthesized mainly in melanocytes located between or below the basal layer of the epidermis and in hair follicles. It is synthesized in melanosomes, which are organelles within melanocytes. These melanosomes move to nearby keratinocytes through dendrites, and when keratinocytes rise to the outer layer, skin color is displayed. The mechanism by which melanosomes move from melanocytes to keratinocytes is not precisely understood, but it is known that melanosomes move to the dendrites of melanocytes and are then released from them into the extracellular space, followed by membrane fusion and phagocytosis between the melanosomes and keratinocytes. It is known that receptors such as PAR-2 (protease-activated receptor 2), KGFR (Keratinocyte growth factor receptor), and TLRs (Toll-like receptors) are involved in the regulation of phagocytosis of the above melanosomes by keratinocytes.

Melanin synthesis is ultimately synthesized from L-tyrosine through DOPA, DOPAquinone, DOPAchrome, and DHI (5,6-dihydroxyindole). Traditionally, inhibition of melanin synthesis has focused on factors that inhibit the activity of tyrosinase, which is an enzyme that catalyzes the rate-controlling step of the melanin synthesis pathway.

PCT International Publication No. WO2020/153819 discloses a polypeptide having an activity of inhibiting the activity of tyrosinase, a key enzyme in the melanin synthesis pathway, and its use for skin whitening. Furthermore, Korean Patent No. 10-1869783 describes a peptide having an activity that inhibits melanin production and tyrosinase activity, and its use for whitening.

Previous skin whitening agent development has focused on inhibiting the activity of tyrosinase, a key enzyme in melanin synthesis. However, to achieve synergistic skin whitening effects through diverse sites of action, the development of skin whitening agents with distinct mechanisms and sites of action is necessary.

### [Prior Art Documents]

### [Patent Documents]

WO2020/153819

Korean Patent No. 10-1869783

### [Disclosure]

### [Technical Problem]

The present inventors have conducted research efforts to develop a peptide with more improved skin whitening activity, and as a result, they have experimentally demonstrated that the novel peptide developed by the present inventors has very excellent inhibitory activity on the uptake of melanosomes into keratinocytes, and have confirmed that the peptide of the present invention can be used as an active ingredient of a skin whitening agent, thereby completing the present invention.

Accordingly, one object of the present invention is to provide a novel peptide having skin whitening activity.

Another object of the present invention is to provide a composition for skin whitening comprising a peptide having the aforementioned activity as an active ingredient.

Yet another object of the present invention is to provide a pharmaceutical composition for the prevention or treatment of hyperpigmentation disorders, comprising a peptide having the aforementioned activity as an active ingredient.

Still yet another object of the present invention is to provide a cosmetic composition for skin whitening, comprising a peptide having the aforementioned activity as an active ingredient.

### [Technical Solution]

To achieve the above objects,
one aspect of the present invention provides a peptide comprising the amino acid sequence of SEQ ID NO: 1.

Another aspect of the present invention provides a composition for skin whitening comprising the peptide as an active ingredient.

Yet another aspect of the present invention provides a pharmaceutical composition for the prevention or treatment of hyperpigmentation disorders comprising the peptide as an active ingredient.

Still yet another aspect of the present invention provides a cosmetic composition for skin whitening comprising the peptide as an active ingredient.

Hereinafter, the present invention will be described in detail.

### 1. Peptide and its activity

According to one aspect of the present invention, a peptide comprising an amino acid sequence disclosed in SEQ ID NO: 1 is provided.
[Amino acid sequence of SEQ ID NO: 1]
KLKKDRMQRS (Lys-Leu-Lys-Lys-Asp-Arg-Met-Gln-Arg-Ser)

As used herein, the term "peptide" means a linear molecule formed by amino acid residues being linked to each other by peptide bonds.

The peptide comprising the amino acid sequence of SEQ ID NO: 1 of the present invention may be used without modification, but a variant or fragment of amino acids having a different sequence by deletion, insertion, substitution, or a combination thereof of amino acid residues may be used within a range that does not affect the original activity of the peptide, for example, the activity of skin whitening.

The peptide of the present invention can be modified by phosphorylation, sulfation, acrylation, glycosylation, methylation, farnesylation, etc., within a range that does not change its activity.

The peptide of the present invention includes a peptide comprising an amino acid sequence substantially identical to a peptide comprising the amino acid sequence of SEQ ID NO: 1, and a variant or active fragment thereof. The substantially identical amino acid sequence refers to an amino acid sequence having a sequence identity of at least 75%, for example, at least 80%, at least 85%, at least 90%, at least 95%, or at least 97%, with the amino acid sequence of SEQ ID NO: 1. In addition, the peptide may additionally include a targeting sequence, a tag, a labeled residue, or an amino acid sequence manufactured for a specific purpose to increase half-life or peptide stability.

The peptide of the present invention may be modified at the N-terminus and/or C-terminus to select a portion of the amino acid sequence and enhance its activity. Such N-terminal and/or C-terminal modifications can significantly enhance the stability of the peptide of the present invention, and, for example, increase the half-life of the peptide when administered in vivo. The term "stability" above is meant to include not only stability in vivo, which protects the peptide of the present invention from attack by in vivo protein cleavage enzymes, but also storage stability (e.g., room temperature storage stability).

The N-terminal modification may be one in which a protecting group, selected from the group consisting of an acetyl group, a fluoreonylmethoxycarbonyl group, a formyl group, a palmitoyl group, a myristyl group, a stearyl group, and polyethylene glycol (PEG), is attached to the N-terminus of the peptide. The above C-terminal modification may be, but is not limited to, one in which a hydroxyl group (-OH), an amino group (-NH2), an azide (-NHNH2), etc. are attached to the C-terminus of the peptide.

The peptide of the present invention can be prepared by various methods widely known in the art to which the present invention pertains. For example, the peptide of the present invention can be prepared by chemical synthesis methods known in the art, particularly solid-phase synthesis techniques (Merrifield, J. Amer. Chem. Soc. 85: 2149-54 (1963); Stewart, et al., Solid Phase Peptide Synthesis, 2nd. ed., Pierce Chem. Co.: Rockford, 111(1984)) or liquid-phase synthesis techniques (US Patent No. 5,516,891).

The peptide of the present invention has skin whitening activity.

In one embodiment, the peptide of the present invention has the activity of inhibiting the absorption (uptake) of melanosomes in keratinocytes.

Melanosomes produced in melanocytes migrate from melanocytes to keratinocytes. This migration of melanosomes into keratinocytes involves the release of melanosomes from melanocytes and the incorporation of melanosomes into keratinocytes. This incorporation of melanosomes can be alternatively expressed as the absorption (uptake) of melanosomes by keratinocytes or the phagocytosis of melanosomes by keratinocytes.

PAR2 (protease activated receptor 2), TLR3 (Toll-like receptor 3), and KGFR (keratinocyte growth factor receptor) are involved in the process of uptake of melanosomes released from melanocytes by phagocytosis of keratinocytes.

In one embodiment, the peptide of the present invention has an activity of inhibiting the expression of the TLR3 (Toll-like receptor 3) gene or the KGFR (keratinocyte growth factor receptor) gene in keratinocytes.

The TLR3 protein is known to be a receptor protein that recognizes pathogens and plays a crucial role in activating innate immunity. It is known that stimulation of the TLR3 protein in keratinocytes induces enhanced melanosome absorption (J Dermatol Sci. 2019 Dec;96(3):168-177), and it is also known that the TRR3 protein in melanocytes promotes the movement of melanosomes into keratinocytes (Int. J. Mol. Sci. 2020, 21, 9769).

The KGFR is a tyrosine kinase receptor that is expressed in many types of epithelial cells and is activated by four known ligands, FGF-1, FGF-3, FGF-7, and FGF-10, and is known to be involved in the proliferation, differentiation, and wound healing of epithelial cells. In addition, KGFR is also known to promote the migration of melanosomes to keratinocytes (J Invest Dermatol. 2005 Dec;125(6):1190-9., Journal of Investigative Dermatology Vol 128, Issue 3, March 2008, p. 558-567).

As described above, the peptide of the present invention can exhibit skin whitening activity and preventive or therapeutic effects on hyperpigmentation disorders through melanosome absorption inhibition activity.

### 2. Composition for skin whitening and composition for preventing, treating or improving hyperpigmentation disorders

In another aspect of the present invention, a composition for skin whitening is provided, comprising a peptide comprising the amino acid sequence of SEQ ID NO: 1 as an active ingredient.

The peptide comprising the amino acid sequence of SEQ ID NO: 1 of the present invention has skin whitening activity through the mechanism described above.

In another aspect of the present invention, the present invention provides a pharmaceutical composition for the prevention or treatment of hyperpigmentation disorders, comprising a peptide comprising the amino acid sequence of SEQ ID NO: 1 as an active ingredient.

In one embodiment, a pharmaceutical composition comprising the peptide as an active ingredient inhibits melanosome absorption in keratinocytes.

In one embodiment, a pharmaceutical composition comprising the peptide as an active ingredient inhibits the expression of the TLR3 (Toll-like receptor 3) gene or the KGFR (keratinocyte growth factor receptor) gene in keratinocytes.

The term "hyperpigmentation disorders" in this specification refers to disorders caused by excessive increase in the amount of melanin in the skin. Thus, skin color darkens due to an excessive increase in melanin levels. Causes of hyperpigmentation disorders include, but are not limited to, exposure to sunlight, skin inflammation (post-inflammatory hyperpigmentation) such as acne, trauma, hormonal imbalances, and medications.

In one embodiment, the hyperpigmentation disease may be melasma, freckles, age-related pigmentation, solar lentigo, or post-inflammatory hyperpigmentation of the skin.

The pharmaceutical composition of the present invention may comprise a therapeutically effective amount of a peptide comprising the amino acid of SEQ ID NO: 1 of the present invention.

The term "therapeutically effective amount" means an amount sufficient for the peptide, which is an active ingredient of the pharmaceutical composition of the present invention, to achieve its activity or efficacy, for example, an amount sufficient to achieve the efficacy of treating or preventing hyperpigmenatation disorders.

The term "prevention" as used herein means reducing the risk of developing a disease or disorder, and means any action that inhibits or delays the onset of a disease by preventing the disease or one or more of its clinical symptoms from progressing.

The term "treatment" as used herein means alleviating a disease or disorder, and includes any action that improves or beneficially alters the symptoms of a disease by arresting or reducing the progression of the disease or one or more of its clinical symptoms.

In the present invention, the prevention or treatment of hyperpigmentation disorders may be to remove the cause of hyperpigmentation in the skin or to inhibit the progression to hyperpigmentation, and specifically, to inhibit the incorporation of melanosomes into keratinocytes.

The pharmaceutical composition of the present invention may include a pharmaceutically acceptable carrier.

The pharmaceutically acceptable carrier is one commonly used in formulations, and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil.

The pharmaceutical composition of the present invention may additionally include, in addition to the above ingredients, a lubricant, a wetting agent, a sweetener, a flavoring agent, an emulsifier, a suspending agent, a preservative, etc., but is not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention can be administered by any suitable route for treating hyperpigmentation disorders, for example, it can be administered orally or parenterally, and in the case of parenteral administration, it can be administered by intravenous injection, subcutaneous injection, intramuscular injection, intraperitoneal injection, topical administration, transdermal administration, etc. Since the pharmaceutical composition of the present invention has an activity for preventing or treating hyperpigmentation disorders of the skin, it is preferable to apply it by topical administration, such as applying it to the skin.

The dosage of the pharmaceutical composition may be, but is not limited to, 0.0001 µg to 100 mg, 0.001 µg to 100 mg, 0.01 µg to 100 mg, 0.1 µg to 100 mg or 1.0 µg to 1000 mg per day, and may be prescribed in various ways depending on factors such as formulation method, administration method, patient age, weight, sex, pathological condition, food, administration time, administration route, excretion rate, and reaction sensitivity.

The pharmaceutical composition of the present invention can be prepared in a unit dose form or can be prepared by inserting it into a multi-dose container by formulating it using a pharmaceutically acceptable carrier and/or excipient according to a method that can be easily performed by a person having ordinary skill in the art to which the present invention pertains. In this case, the formulation may be in the form of a solution, suspension or emulsion in an oil or aqueous medium, or in the form of an extract, powder, granules, tablet or capsule, and may additionally include a dispersing agent or stabilizer.

The pharmaceutical composition of the present invention may be a topical skin preparation. The topical skin preparation is a preparation that can be applied to the outside of the skin. When the pharmaceutical composition of the present invention is used as a topical skin preparation, it may be applied to the skin, specifically to an area of the skin where hyperpigmentation has occurred. The above topical skin preparation may be a cream, gel, ointment, skin emulsifier, skin suspension, transdermal patch, drug-containing bandage, lotion, or a combination thereof. The topical skin preparation may be appropriately mixed with ingredients commonly used in topical skin preparations such as cosmetics or medicines, for example, aqueous ingredients, oily ingredients, powder ingredients, alcohols, moisturizers, thickeners, UV absorbers, whitening agents, preservatives, antioxidants, surfactants, fragrances, colorants, various skin nutrients, or a combination thereof, as needed. The above topical skin preparation may also appropriately contain metal sequestrants such as sodium edetate, trisodium edetate, sodium citrate, sodium polyphosphate, sodium metaphosphate, and gluconic acid; caffeine, tannin, verapamil, licorice extract, glabridin, hot water extract of the fruit of *Pseudocydonia sinensis;* various herbal medicines; tocopheryl acetate, glycyrrhizic acid, tranexamic acid and derivatives or salts thereof; and vitamin C, magnesium ascorbic acid phosphate, ascorbic acid glucoside, arbutin, kojic acid, and sugars such as glucose, fructose and trehalose.

In another aspect of the present invention, the present invention provides cosmetic compositions for skin whitening comprising a peptide comprising the amino acid sequence of SEQ ID NO: 1 as an active ingredient.

In one embodiment, a cosmetic composition comprising the peptide of the present invention as an active ingredient inhibits the absorption of melanosomes in keratinocytes.

In one embodiment, a cosmetic composition comprising the peptide of the present invention as an active ingredient inhibits the expression of the TLR3 (Toll-like receptor 3) gene or the KGFR (keratinocyte growth factor receptor) gene in keratinocytes.

The cosmetic composition may be prepared in any formulation commonly manufactured in the technical field to which the present invention pertains, and may be a topical skin preparation. For example, it may be formulated as a solution, suspension, emulsion, paste, gel, cream, lotion, powder, soap, surfactant-containing cleanser, oil, powder foundation, emulsion foundation, wax foundation, and spray, but is not limited thereto.

The cosmetic composition may be prepared in various forms such as a solution, sol-gel, emulsion, oil, wax, aerosol, etc., such as a flexible toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, a gel, etc., but is not limited thereto.

The cosmetic composition of the present invention may include other additives such as excipients and carriers, and it is possible to apply and mix as much as necessary the usual ingredients mixed in general skin cosmetics.

When the formulation of the cosmetic composition is in the form of a paste, cream or gel, as a carrier component, animal oil, vegetable oil, wax, paraffin, starch, tragacanth, cellulose derivatives, polyethylene glycol, silicone, bentonite, silica, talc or zinc oxide may be used.

When the formulation of the cosmetic composition is in the form of a powder or spray, as a carrier component, lactose, talc, silica, aluminum hydroxide, calcium silicate or polyamide powder may be used. In particular, in the case of a spray, a propellant such as chlorofluorohydrocarbon, propane/butane or dimethyl ether may be additionally included, but is not limited thereto.

When the formulation of the cosmetic composition is in the form of a solution or emulsion, a solvent, a solubilizer or an emulsifier may be used as a carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the formulation of the cosmetic composition is in the form of a suspension, a liquid diluent such as water, ethanol or propylene glycol, a suspending agent such as ethoxylated isostearyl alcohol, polyoxyethyl sorbitol ester and polyoxyethylene sorbitan ester, microcrystalline cellulose, aluminum metahydroxide, bentonite, agar or tragacanth, etc. can be used as a carrier component.

When the formulation of the above cosmetic composition is a surfactant-containing cleansing, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, fatty alcohol, fatty acid glyceride, fatty acid diethanolamide, vegetable oil, lanolin derivative, or ethoxylated glycerol fatty acid ester may be used as a carrier component.

When the formulation of the cosmetic composition is a hair shampoo, base ingredients for forming a shampoo, such as a thickener, a surfactant, a viscosity modifier, a moisturizer, a pH adjuster, a preservative, and essential oils, may be mixed. CDE may be used as the thickener, LES, which is an anionic surfactant, and coco betaine, an amphoteric surfactant, may be used as the surfactant, polyquaternary may be used as the viscosity modifier, glycerin may be used as the moisturizer, and citric acid or sodium hydroxide may be used as the pH adjuster. As the preservative, grapefruit extract, etc. can be used, and in addition, essential oils such as cedarwood, peppermint, and rosemary, as well as silk amino acids, pentaol, or vitamin E can be added.

The ingredients included in the cosmetic composition may further include, in addition to the peptide of the present invention as an active ingredient and the carrier component, ingredients commonly used in cosmetic compositions, such as conventional auxiliary agents such as antioxidants, stabilizers, solubilizers, vitamins, pigments, and fragrances, but are not limited thereto.

The peptide of the present invention may be included in the above-described composition, pharmaceutical composition or cosmetic composition at a concentration of 0.01 µM to 1000 µM, specifically, the peptide of the present invention may be included at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

### 3. Use of peptide of the present invention

In another aspect of the present invention, a peptide comprising the amino acid sequence of SEQ ID NO: 1 is provided for use in skin whitening or for preventing, treating or improving hyperpigmentation disorders.

In another aspect of the present invention, a skin whitening method is provided, comprising administering a peptide comprising the amino acid sequence of SEQ ID NO: 1 or a composition comprising the peptide to a subject in need of skin whitening.

In another aspect of the present invention, a method for preventing or treating hyperpigmenatation disorders is provided, comprising administering a peptide comprising the amino acid sequence of SEQ ID NO: 1 or a composition comprising the peptide to a subject in need of prevention or treatment of hyperpigmentation disorders.

In another aspect of the present invention, there is provided the use of a peptide comprising the amino acid sequence of SEQ ID NO: 1 for the preparation of a medicament for the prevention or treatment of hyperpigmentation disorders.

In another aspect of the present invention, the use of a peptide comprising the amino acid sequence of SEQ ID NO: 1 for the manufacture of cosmetics for skin whitening is provided.

### [Advantageous Effects]

The peptide of the present invention inhibits the melanosome absorption in keratinocytes. Therefore, the peptide of the present invention can be used as an active ingredient in drugs for the treatment or prevention of hyperpigmentation disorders caused by excessive deposition of melanosomes, or as an effective ingredient in cosmetics for skin whitening.

However, the effects of the present invention are not limited to the effects mentioned above, and other effects not mentioned will be clearly understood by those skilled in the art from the description below.

### [Description of Drawings]

FIG. 1 shows the results of measuring the absorption rate of melanosomes isolated from HaCaT cells and melanosomes treated with the peptide of the present invention or the positive control ENMD-1068.
FIG. 2 shows the results of measuring the mRNA level of the TLR3 gene after treating HaCaT cells with Poly(I:C), which is a synthetic TLR3 agonist, and then treating them with the peptide of the present invention. The band values are calculated by comparing the densitometry results of the TLR3 gene bands with the densitometry results of the GAPDH band, which is the loading control, and then showing the relative expression level with the control as 1.
FIG. 3 shows the results of measuring the mRNA level of KFGR after treating HaCaT cells with the peptide of the present invention. The band values are calculated by comparing the densitometry results of the KFGR gene bands with the densitometry results of the GAPDH band, which is the loading control, and then showing the relative expression level with the control as 1.
FIG. 4A is a confocal microscopic photograph showing the fluorescence of microspheres introduced into the cells after treating HaCaT cells with microspheres and treating them with Poly(I:C) and the peptide of the present invention.
FIG. 4B is a graph showing the fluorescence values of microspheres introduced into cells in each control and experimental group.

### [Mode For Carrying Out The Invention]

Hereinafter, the present invention will be described in detail by way of examples. However, the following examples specifically illustrate the present invention, and the content of the present invention is not limited by the following examples.

### Preparation Example 1: Preparation of peptide and peptide complex

A peptide having the amino acid sequence of SEQ ID NO: 1 shown in Table 1 below was synthesized using an automatic peptide synthesizer (Milligen 9050, Millipore, USA), and the synthesized peptide was purified using C18 reverse-phase high-performance liquid chromatography (HPLC) (Waters Associates, USA). The column used was ACQUITY UPLC BEH300 C18 (2.1 mm X 100 mm, 1.7 µm, Waters Co, USA).

**[Table 1]**

| SEQ ID NO | Amino acid sequence of peptide |
|---|---|
| 1 | KLKKDRMQRS (Lys-Leu-Lys-Lys-Asp-Arg-Met-Gln-Arg-Ser) |

The efficacy of the peptide of SEQ ID NO: 1 prepared above was evaluated through the following experiment.

### Experimental Example 1: Analysis of absorption by melanosome phagocytosis

It was confirmed whether the peptide prepared in Preparation Example 1 inhibits the absorption (uptake) of melanosomes by phagocytosis in keratinocytes.

HaCaT cells (human keratinocyte cell line) were seeded at a density of 3 x 10⁵ cells/well in 6-well cell culture plates and cultured in DMEM (Dulbecco's Modified Eagle's Medium) medium containing 10% FBS for 24 hours. Subsequently, the cells were replaced with serum-free DMEM medium, and the peptides of Preparation Example 1 were added to the serum-free DMEM medium to prepare peptide solutions at concentrations of 50µM, 100 µM and 200 µM, which were then added to the cultured cells. At this time, the negative control was a group without peptide treatment, and the positive control was treated with 500 µM ENMD-1068 (6-Amino-1-[4-(3-methyl-1-oxobutyl)-1-piperazinyl]-1-hexanone hydrochloride, Sigma-Aldrich). Subsequently, after culturing in a CO₂ incubator at 37°C for 24 hours, 10 µg/ml melanosomes isolated from B16F10 cells (mouse melanoma cell line) were additionally added to the cultured cells, excluding the control group. Subsequently, after culturing in a CO₂ incubator at 37°C for 48 hours, HaCaT cells were harvested after washing three times with PBS. HaCaT cell pellets were collected using a centrifuge, dissolved in 1 M NaOH, and dispensed into 96-well plates. To measure melanin content, absorbance at a wavelength of 490 nm was measured using a spectrophotometer.

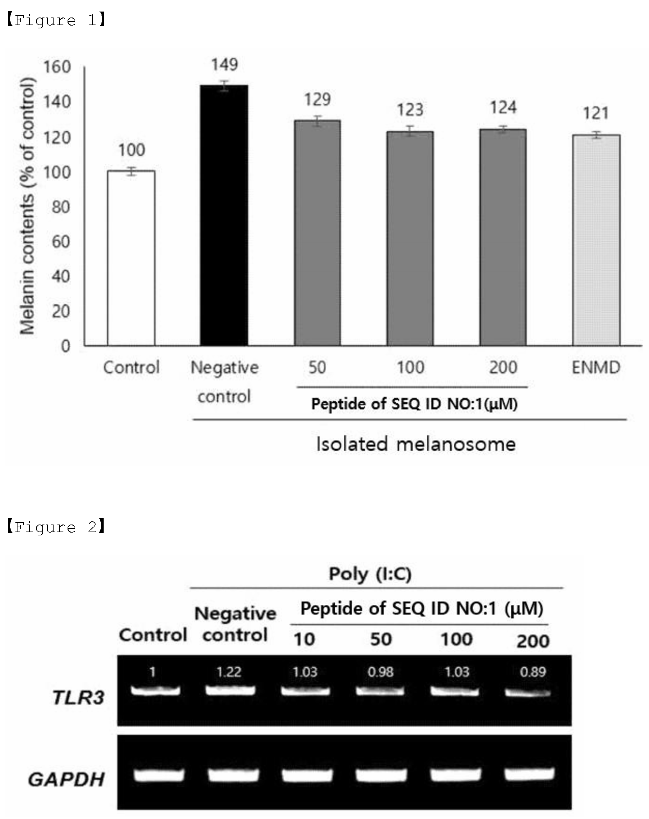

As a result of the experiment, as can be seen from the melanin content analysis results in FIG. 1, it was confirmed that the absorption (uptake) of melanosomes in HaCaT cells was inhibited by the peptide of Preparation Example 1.

### Experimental Example 2: Expression analysis of TLR3, a gene involved in melanosome phagocytosis

The effect of the peptide prepared in Preparation Example 1 on the expression of the TLR3 (Toll-like receptor 3) gene, which is a gene involved in melanosome phagocytosis, was evaluated.

HaCaT cells were seeded at a density of 3 x 10⁵ cells/well in 6-well plates, replaced with DMEM medium containing 2% FBS, and cultured in a CO₂ incubator at 37°C for 24 hours. The peptide of Preparation Example 1 was added to the DMEM medium containing 2% FBS to prepare peptide solutions at concentrations of 10 µM, 50 µM, 100 µM, and 200 µM, and these peptide solutions were added to the cells and treated for 30 minutes. Subsequently, 30 µg/mL of poly(I:C) (Polyinosinic-Polycytidylic acid), which is a TLR3 agonist, was additionally added, and cultured in a CO₂ incubator at 37°C for 24 hours. After washing twice with PBS, RNA was isolated using easy blue (iNtRON, Cat. No.: 17061, Korea). The amount of isolated RNA was quantified, and 2,000 ng of RNA was aliquoted per tube, and cDNA was synthesized using a cDNA synthesis kit (enzynomics, Cat. No.: RT200, Korea). PCR was performed using primers targeting the TLR3 gene in Table 2 below and a PCR kit (enzynomics, Cat. No.: P581T, Korea). The PCR products were then electrophoresed on a 1.2% agarose gel, and bands were detected and analyzed using a Bio-Rad gel image system.

As a result of the experiment, as shown in FIG. 2, it was confirmed that when HaCaT cells were treated with Poly (I:C), which is an agonist of TLR3, the expression level of the TLR3 gene increased, and the increased expression level of the TLR3 gene was suppressed by treating with the peptide of Preparation Example 1.

### Experimental Example 3: Expression analysis of KGFR, a gene involved in melanosome phagocytosis

The effect of the peptide of SEQ ID NO: 1 prepared in Preparation Example 1 on the expression of the melanosome phagocytosis-related gene KGFR (Keratinocyte growth factor receptor) gene was evaluated.

HaCaT cells were seeded at a density of 3 x 10⁵ cells/well in 6-well plates and cultured in DMEM medium containing 10% FBS for 24 hours. After replacing the cells with serum-free DMEM medium, they were cultured in a CO₂ incubator at 37°C for 24 hours. Peptide solutions at concentrations of 10 µM, 50 µM, 100 µM, and 200 µM were prepared by adding the peptides of Preparation Example 1 to serum-free DMEM medium, and dispensed onto the cells and further cultured in a CO₂ incubator at 37°C for 6 hours. After washing twice with PBS, RNA was isolated using easy blue (iNtRON, Cat. No.: 17061, Korea). The amount of isolated RNA was quantified, and cDNA was synthesized using an RNA and cDNA synthesis kit (enzynomics, Cat. No.: RT200, Korea). PCR was performed using primers targeting the KGFR gene in Table 2 below and a PCR kit (enzynomics, Cat. No.: P581T, Korea). The PCR products were then electrophoresed on a 1.2% agarose gel, and bands were detected and analyzed using a Bio-Rad gel image system.

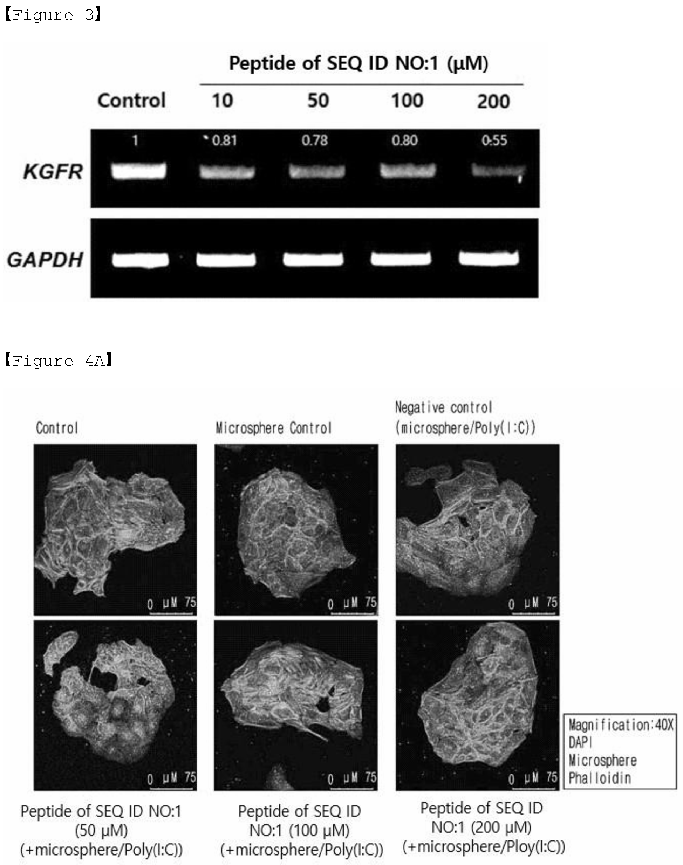

As a result of the experiment, as shown in FIG. 3, it was confirmed that the expression level of the KGFR gene was reduced in a concentration-dependent manner by peptide treatment of Preparation Example 1 in HaCaT cells.

### Experimental Example 4: Analysis of Poly (I:C)-induced phagocytosis - Analysis of microsphere absorption

The effect of the peptide prepared in Preparation Example 1 on Poly (I:C)-induced melanosome phagocytosis was evaluated through a microsphere absorption (microsphere uptake) experiment.

HaCaT cells were seeded at a density of 3 x 10⁴ cells/well in 6-well plates and cultured using DMEM medium containing 10% FBS in a CO₂ incubator at 37°C for 24 hours. After replacing the medium with serum-free DMEM medium, the peptides of Preparation Example 1 were added to the serum-free DMEM medium to prepare peptide solutions at concentrations of 50 µM, 100 µM, and 200 µM, and the cells were pretreated with these peptide solutions for 1 hour. After treatment with 30 µg/mL of Poly (I:C) (Polyinosinic-Polycytidylic acid) and microspheres (FluoSpheres^{™}, diameter: 1 µm), the cells were cultured in a CO₂ incubator at 37°C for 24 hours. The negative control was treated with Poly (I:C) and microspheres without peptide treatment. Cells were fixed by treatment with 4% formaldehyde for 30 minutes. After treatment with phalloidin iFluor 488 reagent (Abcam, UK), the cells were reacted for 1 hour. After treatment with DAPI-containing mounting medium (Invitrogen, USA), confocal microscopy (400x magnification) was used for observation. The fluorescence value of microspheres internalized into the cells was analyzed using the Image J program.

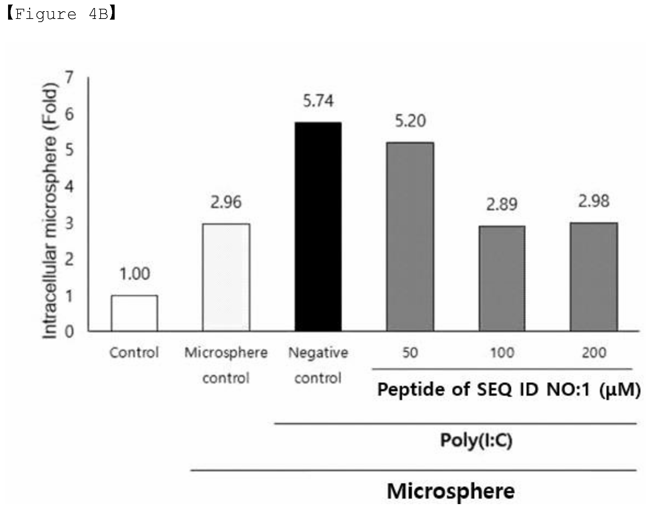

As a result of the experiment, as shown in FIGS. 4A and 4B, it was confirmed that the absorption of microspheres increased by Poly (I:C) treatment, and the increased absorption of microspheres was reduced by the peptide treatment of Preparation Example 1.

### PCR primer sequence list (Table 2)

**[Table 2]**

| Primer name | Sequence (5' → 3') | SEQ ID NO |
|---|---|---|
| TLR3 Forward | (5') GCC GTC TAT TTG CCA CAC AC (3') | 2 |
| TLR3 Reverse | (5') GCA GTC AGC AAC TTC ATG GC (3') | 3 |
| KGFR Forward | (5') TGA CCA AAC GTA TCC CCC TG (3') | 4 |
| KGFR Reverse | (5') GGT GTC TGC CGT TGA AGA GA (3') | 5 |
| GAPDH Forward | (5') GGA GCC AAA AGG GTC ATC AT (3') | 6 |
| GAPDH Reverse | (5') GTG ATG GCA TGG ACT GTG GT (3') | 7 |

Although representative embodiments of the present application have been described above as examples, the scope of the present application is not limited to the specific embodiments described above, and a person with ordinary knowledge in the relevant field will be able to make appropriate changes within the scope described in the claims of the present application.

## Claims

1. A peptide comprising an amino acid sequence of SEQ ID NO: 1.

2. A composition for skin whitening comprising the peptide of claim 1 as an active ingredient.

3. A pharmaceutical composition for the prevention or treatment of hyperpigmentation disorders, comprising the peptide of claim 1 as an active ingredient.

4. The pharmaceutical composition for the prevention or treatment of hyperpigmentation disorders of claim 3, wherein the peptide inhibits the absorption of melanosomes in keratinocytes.

5. The pharmaceutical composition for the prevention or treatment of hyperpigmentation disorders of claim 4, wherein the peptide inhibits the expression of (i) TLR3 (Toll-like receptor 3) gene, or (ii) KGFR (keratinocyte growth factor receptor) gene, in keratinocytes.

6. The pharmaceutical composition for the prevention or treatment of hyperpigmentation disorders of claim 3, wherein the hyperpigmentation disorders are disorders that occur when the amount of melanin in the skin increases excessively.

7. The pharmaceutical composition for the prevention or treatment of hyperpigmentation disorders of claim 3, wherein the hyperpigmenatation disorders are melasma, freckles, age-related pigmentation, solar lentigo, or post-inflammatory hyperpigmentation of skin.

8. The pharmaceutical composition for the prevention or treatment of hyperpigmentation disorders of claim 3, wherein the pharmaceutical composition is a topical skin preparation.

9. A cosmetic composition for skin whitening comprising the peptide of claim 1 as an active ingredient.

10. The cosmetic composition for skin whitening of claim 9, wherein the peptide inhibits the absorption of melanosomes in keratinocytes.

11. The cosmetic composition for skin whitening of claim 10, wherein the peptide inhibits the expression of (i) TLR3 (Toll-like receptor 3) gene, or (ii) KGFR (keratinocyte growth factor receptor) gene, in keratinocytes.

12. The cosmetic composition for skin whitening of claim 9, wherein the cosmetic composition is a topical skin preparation.

13. The cosmetic composition for skin whitening of claim 12, wherein the cosmetic composition is at least one formulation selected from the group consisting of a solution, a suspension, an emulsion, a paste, a gel, a cream, a lotion, a powder, a soap, a surfactant-containing cleansing, an oil, a powder foundation, an emulsion foundation, a wax foundation, and a spray.

14. A method for preventing or treating hyperpigmenatation disorders, comprising administering the peptide of claim 1 or a composition comprising the peptide to a subject in need of prevention or treatment of hyperpigmentation disorders.

15. The method for preventing or treating hyperpigmenatation disorders of claim 14, wherein the hyperpigmenatation disorders are melasma, freckles, age-related pigmentation, solar lentigo, or post-inflammatory hyperpigmentation of skin.
